# EUROPEAN PATENT SPECIFICATION

(11) **EP 0 991 691 B1**
(45) Date of publication and mention of the grant of the patent: **15.01.2003**
(21) Application number: 98932264.9
(22) Date of filing: 16.06.1998
(51) Int. Cl.: C08G 63/52, A61K 7/48, A61K 31/215

(54) **POLYMER COMPRISING UNSATURATED ESTER UNITS AND PHARMACEUTICAL AND COSMETIC COMPOSITIONS THEREOF**
POLYMER ENTHALTEND EINHEITEN UNGESÄTTIGTER POLYESTER SOWIE DARAUS HERGESTELLTE PHARMAZEUTISCHE UND KOSMETISCHE ZUSAMMENSETZUNGEN
POLYMERE RENFERMANT DES MOTIFS ESTER INSATURES, COMPOSITIONS PHARMACEUTIQUES ET COSMETIQUES REALISEES A PARTIR DE CE POLYMERE

(30) Priority: 19.06.1997 EP 97304295
(43) Date of publication of application: 12.04.2000
(73) Proprietor: UNICHEMA CHEMIE B.V., 2802 BE Gouda (NL)
(72) Inventor: LINDNER, Nigel, Northamptonshire NN14 4BD (GB); BOSLEY, John, Northamptonshire NN14 3JT (GB); MACRAE, Alasdair, Newton Blossomville, Bedford MK43 8AN (GB); SVENSSON, Ingemar, S-226 57 Lund (SE)
(74) Representative: Humphries, Martyn
(86) International application number: GB9801753
(87) International publication number: WO98058010

(56) References cited:
- EP-A- 0 336 880
- GB-A- 665 595
- GB-A- 2 089 824
- US-A- 4 661 519
- Database STN, file CA Abs. No. 113:85279 &J.Am.Chem.Soc. (1990),112(15),5851-4 Jayasuriya,N. et al. "supramolecular surfactants..." together with Database STN file reg. Reg No's128661-17-6 and 128661-12-1 XP002074857
- Database STN, file CA Abs. No. 82:58643 &Farbe Lack (1974), 80(11),1028-9 Van Thiel, J.M. et al. Applications of dicarboxylic..." together with Database STN file Reg Reg. No. 13481-097-5 XP002074858

## Description

### Field of the Invention

This invention relates to certain polymeric molecules, compositions comprising polymeric molecules, a method of making said compositions, and to methods of treating a human subject.

### Background of the Invention

Saturated dicarboxylic ("dioic") acids and their derivatives are compounds which are noted for their antimicrobial activity and for their beneficial effects on human skin. In particular the C₉ saturated dioic acid ("azelaic" acid), having the formula COOH-(CH₂)₇-COOH, is frequently cited as being effective in the treatment of acne and other skin conditions, and in the lightening of skin. Dioic acid derivatives which are suggested to be useful include salts, esters, amides, and mercapto-compounds (e.g. US 4,818,768, US 4,292,326, EP 0,297,436, EP 0,305,407, JP 58-170713).

The prior art teaches that conditions susceptible to treatment with dioic acids include: acne (US 4.386. 104); wrinkles (EP 0,336,880); malignant melanoma (US 4,818,768); dermatoses (EP 0,229,654); hyperpigmentary dermatosis and eczema (US 4,292,326); rosacea (EP 0,890,308); lentigo (JP 91024412) and seborrhoea (DE 3133425) and impetigo.

WO 94/07837 teaches that unsaturated dioic acids and derivatives thereof are generally more effective than their saturated counterparts, having greater anti-microbial and skin-lightening activity. WO 94/07837 also discloses a microbial method of making unsaturated dioic acids. There is no disclosure of polymers comprising unsaturated dioic acids.

WO 94/12652 discloses a method of producing a polyester, comprising as a repeating unit "at least one aliphatic dicarboxylic acid". The document states (page 5) that: "suitable aliphatic dicarboxylic acids include those of formula HO₂C-R⁵CO₂H wherwn R⁵ is a bond or is a divalent radical defined as for R¹" (R¹ having been previously defined in the document as a "substituted or unsubstituted C₁ to C₁₂ alkyl group optionally having one or more carbon-carbon double bonds..."). The polyesters produced by the process described in WO 94/12652 are said to "find uses as shaped articles and foams, particularly for motor vehicles". There is no suggestion of the use of polymers comprising dioic acids in any other technical fields.

### Summary of the Invention

In a first aspect the invention provides a polymeric molecule comprising a plurality of repeat units having the structure -(O₂C-R-CO₂)-, wherein R is a substituted or unsubstituted, C_{16:1}, C_{16:2}, C_{16:3} or a C_{20:1} hydrocarbon moiety, and wherein the molecule is a copolymer of a dioic acid esterified with glycerol, butanediol or a C₆- C₁₀ polyol. It is preferred that the polymer is a lipase-degradable polyester, which upon exposure to a lipase releases a compound having a beneficial effect on a human subject. The released. compound will therefore be a substituted or unsubstituted, unsaturated dioic acid.

In a second aspect, the invention provides a composition suitable for topical application to the human body, the composition comprising: a polymeric molecule having a plurality of repeat units having the structure -(O₂C-R-CO₂)-, wherein R is a substituted or unsubstituted, unsaturated hydrocarbon radical having 10 or more carbon atoms; and an . acceptable carrier substance. Preferably the composition comprises a polymeric molecule in accordance with the first aspect defined above. Such compositions have unexpected properties and are suitable for a wide range of purposes, discussed further below. be respectively obtained from oleic, linoleic, linolenic or erucic acids).

The preferred average molecular weight of the polymeric molecule will also depend, in part, on the intended use of the composition comprising the polymeric molecule. The lower and upper limits are around 1kDa and 50 kDa respectively. A polymer of around 1kDa molecular weight will have about 3 or 4 repeat units. The generally preferred range is 3.5-10kDa. Polymers of this size are generally liquids and thus more readily processed.

The compositions of the invention have a considerable number of possible uses, all of which are considered as being within a further aspect of the invention. The nature and structure of the polymeric molecules can be tailored to the intended purpose of the composition. Indeed, in some circumstances it will be advantageous if the composition comprises two or more distinct polymeric molecules of the type defined above with complementary characteristics.

The compositions of the invention are formulated for topical application to the human body, advantageously being suitable for application to the skin, scalp or hair. Generally it is envisaged that, as with the compositions disclosed in WO 94/07837, the compositions of the present invention are useful as cosmetic compositions, improving skin quality and complexion, and as pharmaceutical compositions having anti-microbial effects (which can also enhance skin condition). Specifically, the compositions of the invention may be of use in the treatment of: acne; wrinkles; malignant melanoma; demiatoses: hyperpigmentary dermatosis and eczema; rosacea; lentigo, seborrhoea and impetigo; and in the treatment of body malodour. Additionally, the compositions may be used for the lightening of skin. In particular, the compositions are useful as anti-perspirants and/or deodorants.

The compositions may *inter alia*. take the form of gels, creams, lotions, oils or waxes (hard or soft).

Advantageously the composition will be buffered to the range pH 4-7, preferably within the range pH 5.5-6.5, at which pH the dioic acid (or derivative) monomeric units released by the action of a lipase appear to exert optimum anti-microbial effect.

Once applied to the skin, the polymeric molecules present in the composition are subject to degradation mediated by lipases present on the surface of the human body. Generally such lipases are secreted by microorganisms which colonise the skin. Such lipase-mediated degradation leads to the release of dioic acid monomer units (or substituted dervatives thereof) from the polymeric molecule, which monomer units have desirable cosmetic and antimicrobial properties. Thus, there is controlled release of active agent from the polymeric molecule, which occurs most rapidly in response to high concentrations of lipases, which will be present at sites of maximum microbial colonisation (e.g. spots, locally inflamed areas, skin pores).

Most lipases have greater freedom to act at the ends of polymeric molecules rather than in the middle of the polymer chain, where steric considerations can inhibit the action of the enzyme. Accordingly, the concentration of "free ends" available for lipase-mediated degradation is relatively greater for a composition comprising short polymeric molecules than for compositions comprising long polymeric molecules. Also, the lower molecular weight polymers are thought to be less crystalline in structure, and so more accessible to lipases. It will therefore be advantageous for some uses (e.g. as topically-applied cosmetic agents) if compositions in accordance with the invention comprise polymeric molecules with a range of molecular weights, such that smaller polymers will be present for relatively rapid degradation, giving relatively quick release of active agent, whilst larger polymers which are degraded more slowly are included to give a longer lasting supply of active agent. The choice of polymer size can therefore be tailored to suit the intended purpose, and/or frequency of application, of the composition.

The present inventors expect that compositions in accordance with the present invention have surprising properties which make them suitable for many other uses. Suitable compositions can form occlusive, moisture-retaining films when applied to the skin. Thus the compositions are particularly useful in barrier creams, and in "after sun" creams, gels and lotions and as anti-perspirants/deodorants. In addition the compositions may be used to form clear or translucent dermal patches, which can comprise other substances which are desired to be delivered transdermally (e.g. nicotine).

Where it is desired to maximise the occlusive properties of the composition, it will generally be preferred to use a longer chain dioic acid repeating unit and/or a longer chain alcohol, to increase the hydrophobicity of the resulting polymeric molecule.

The occlusive, anti-microbial characteristics of compositions in accordance with the invention renders them ideal for use in dressings to promote wound healing (especially healing of burns, where both moisture-retention and anti-microbial properties are desired). Thus, in another aspect, the invention provides a medical dressing (such as a bandage, plaster, mesh, wound dressing etc.) for application to the skin of a human subject, the dressing comprising a dressing substrate impregnated or coated with a polymeric molecule in accordance with the invention.

Suitable formulations for the compositions of the invention will be apparent to those skilled in the art and include those, for example, taught by US 4,818,768.

In particular it will be preferred that compositions for direct application to the skin will comprise a relatively volatile organic solvent (such as lower alcohols, C₂-C₄). Mixing an organic solvent with the polymeric molecule causes a substantial decrease in the viscosity of the composition, thereby facilitating application of the composition as a liquid or a spray: after application, a relatively volatile organic solvent will evaporate, leaving a film of the polymeric molecule (which may subsequently be hydrolysed by lipases).

The polymeric molecules for inclusion in the composition of the invention may be synthesised by standard chemical methods, or may be produced via biocatalytic methods, as described in the examples below and in WO 94/12652. Suitable lipases are also disclosed therein. Lipases obtainable from *Candida antarctica* are particularly suitable. Biocatalytic methods are generally to be preferred when forming polymers comprising polyols, as the substrate specificity of lipases ensures a greater degree of uniformity of product (with reaction mainly at the primary -OH groups) than that obtainable by conventional chemical methods. In particular, the use of an enzyme avoids excessive cross-linking (with reaction occurring mainly at the primary -OH groups), ensuring an essentially linear, bio-degradable polymer. In contrast, conventional chemical methods can result in excessive cross-linking, leading to highly branched products which tend to precipitate out of the reaction mixture in an uncontrolled manner. In addition, when the polymers are synthesised by use of a lipase, it increases the likelihood that the polymeric product will have the desirable property, in the final composition, of being lipase-degradable.

The invention also includes within its scope the use of the composition defined above as a cosmetic and/or therapeutic composition; and a method of making the composition defined above.

Accordingly the invention provides a method of treating the skin of a human subject, comprising applying to the skin a composition in accordance with the second aspect of the invention, the composition comprising a polymer having a plurality of repeat units having the structure -(O₂C-R-CO₂)-, wherein R is a substituted or unsubstituted, unsaturated hydrocarbon radical having 10 or more carbon atoms, said polymer being lipase-degradable to release an active substance having a beneficial anti-microbial and/or cosmetic effect. Conditions treatable by the method are as explained above. Preferably the method of treating skin will comprise the use of a composition comprising a polymer in accordance with the first aspect of the invention.

The invention also provides a method of making a cosmetic or pharmaceutical composition suitable for topical application to a human subject, the method comprising mixing an effective amount of a polymer having a plurality of repeat units having the structure - (O₂C-R-CO₂)-, wherein R is a substituted or unsubstituted, unsaturated hydrocarbon radical having 10 or more carbon atoms, with an acceptable carrier substance (e.g. aqueous solutions, organic solvents, emollients, emulsifiers, or mixtures of any of the foregoing). Desirably the method will comprise the use of a polymer in accordance with the first aspect of the invention.

In particular, the invention provides a method of making a medical dressing (such as a bandage, wound dressing, plaster, mesh or the like) for application to the skin of a human subject, the method comprising coating or impregnating a suitable dressing substrate with a polymer having a plurality of repeat units having the structure -(O₂C-R-CO₂)-, wherein R is a substituted or unsubstituted, unsaturated hydrocarbon radical having 10 or more carbon atoms, to form a dressing. Desirably the medical dressing will be coated or impregnated with a polymer in accordance with the first aspect of the invention. Once made, the dressing will conveniently be packaged and sterilised (e.g. by irradiation).

In a further aspect the invention provides for use of a lipase-degradable polymer in the manufacture of a pharmaceutical composition for the treatment of a human skin condition (e.g. acne, pimples and the like) caused or exacerbated by, or associated with, microbial growth, the polymer having a plurality of repeat units having the structure -(O₂C-R-CO₂)-, wherein R is a substituted or unsubstituted, unsaturated hydrocarbon radical having 10 or more carbon atoms. Advantageously the polymer will be in accordance with the first aspect of the invention.

The invention will now be further described by way of illustrative example and with reference to the accompanying drawings, in which:

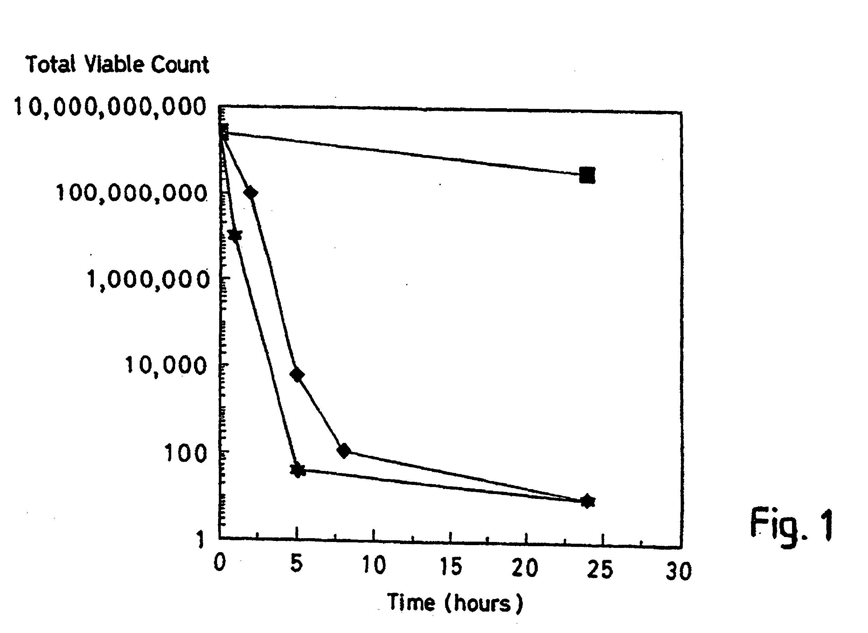

Figure 1 is a graph of total viable count (TVC) against time (hours) for Corynebacteria in the presence of: (i) no added substance (negative control, filled squares): (ii) added C18:1 dioic acid/butanediol polyester (MW 4.800, filled diamond symbols); and (iii) added free C18:1 dioic acid (filled star symbols);

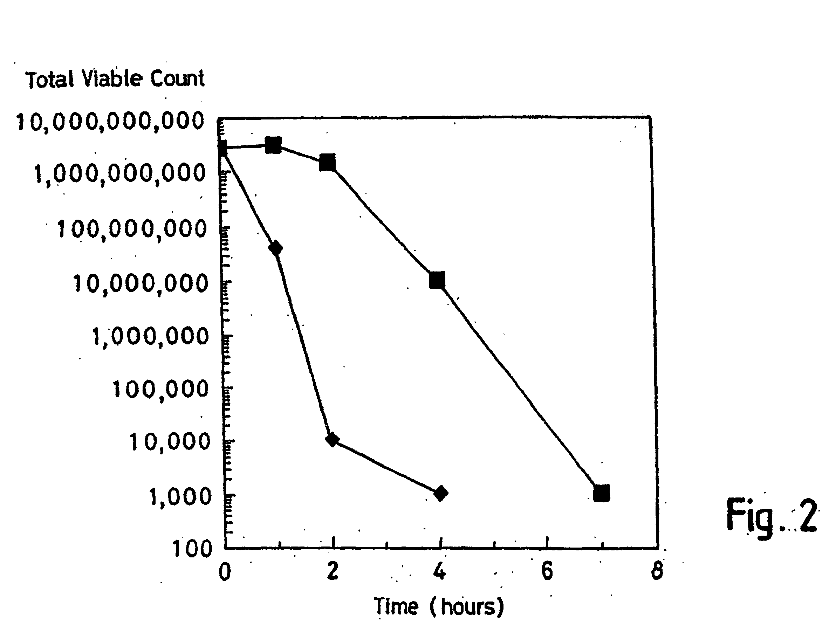

Figure 2 is a graph of total viable count (TVC) against time (hours) for Corynebacteria in the presence of a composition in accordance with the invention with (filled diamond symbol) or without (filled square symbol) exogenously added lipase; and

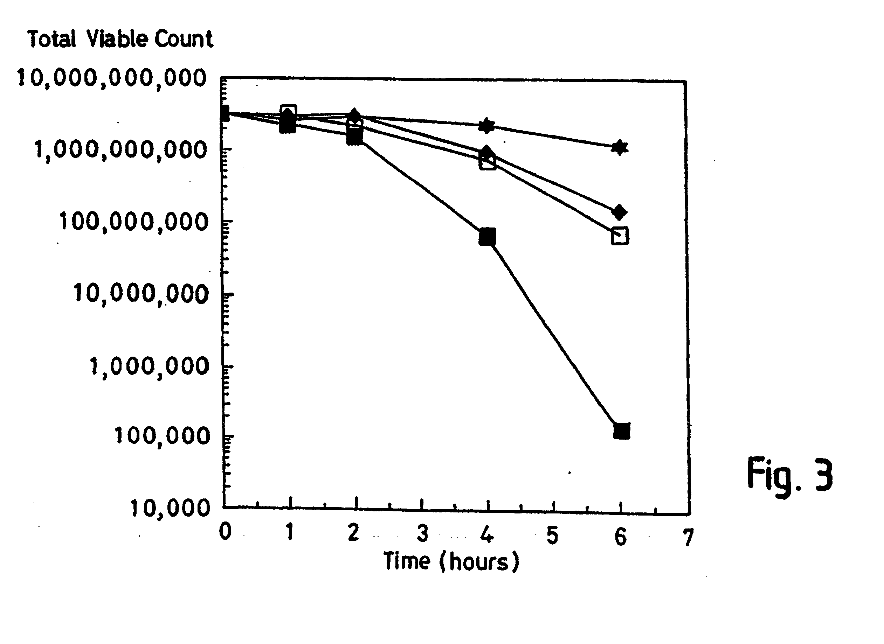

Figure 3 is a graph of total viable count (TVC) against time for Corynebacteria in the presence of one of four different compositions in accordance with the invention, having respective molecular weights of 4,800 (filled squares); 16,600 (filled diamonds); 34,700 (filled stars); or ≈ 4,600 (open squares).

### Example 1 - Synthesis of a co-polymer comprising, as monomers, C_{18:1} dioic acid and butan-1, 4-diol.

### Materials:

9-octadecen-1, 18-dioic acid (C18:1 DCA), ex Unigema, 91.66 g. 1,4-butanediol, ex Fluka, 26.35 g.
Immobilised *Candida antarctica* B lipase, SP 435 (Novo Nordisk, Denmark), 400 mg.

### Reaction:

The substrates were heated to 60°C in a 150 ml glass reactor vessel and stirred at 200 r.p.m. with an overhead stirrer fitted with a Teflon paddle. The dicarboxylic acid melted and dissolved in the butanediol, the reaction was then started by adding the immobilised lipase preparation. After 24 hours reaction the pressure in the head space of the reactor was lowered to 50 mbar, by connecting a vacuum-pump. The water formed during the reaction was then removed under vacuum to force the chemical equilibrium towards ester formation and to increase the molecular weight of the polyester product. After further 24 hours under vacuum the reaction was stopped and the lipase catalyst was removed by vacuum-filtration on a filter paper at 60°C.

### Analyses:

The reaction was followed by taking samples and titrating the free carboxylic acid groups with NaOH-solution using phenolphthalein as indicator. A sample of 0.2-0.4 g was dissolved in 20 ml diethylether/ethanol (1:1) and titrated with 100 mM NaOH in water/ethanol (1:1) until a colour change was noted.

The increase in polymer molecular weight was followed by Gel Permeation-Chromatography (GPC) on one TSK 1000 and one TSK 3000 column from Supelco used in series at 30°C. Solvent used was tetrahydrofuran (THF) at a flow-rate of 1 ml/min. A differential refractive index detector (Gilson) was used as detector. Calibration was done with commercial polystyrene standards except where otherwise indicated. The mass-and number-average molecular weights (Mw and Mn) were calculated by use of a computer program (TURBOGEL™) from P E Nelson.

### Results:

The polyester produced under the conditions described above was an amber-coloured, viscous liquid at room temperature. The Mw was 4800, polydispersity (Mw/Mn) 2.2 and the acid value (mg KOH/g sample) 27.8.

By repeating the reaction with more catalyst and/or longer reaction times higher molecular weights of polyester were achieved. The products were all liquids and showed increasing viscosity with increasing molecular weight. Their properties are summarised in Table 1, below.

**Table 1**

| Polyesters formed from C18:1 DCA and 1,4-butanediol | | | |
|---|---|---|---|
| **Reaction** | **Mw** | **Polydispersity** | **Acid value** |
| 1 | 4800 | 2.2 | 27.8 |
| 2 | 16600 | 2.3 | 22.7 |
| 3 | 34700 | 3.8 | 8.0 |
| 4* | 47400 | 2.0 | 9.0 |

| | | | |
|---|---|---|---|
| * This preparation contained some THF-insoluble material. | | | |

### Example 2

The butanediol monomer was substituted with glycerol and using the 1,3-specific lipase from *Rhizomucor miehei* (Lipozyme ex Novo Nordisk. Denmark). A non-crosslinked polyester containing hydroxyl side groups was formed.

### Materials:

9-octadecen 1,18-dioic acid, ex Unigema, 50.0 g.
glycerol, 99% ex Aldrich, 14.7 g.
distilled water, 645 µl.
Immobilised *Rhizomucor miehei* lipase (Lipozyme IM60), 2.0 g.

### Reaction:

The reaction was performed as described in Example 1, except that the initial vacuum. applied was 100 mbar. This vacuum was gradually lowered during the course of the reaction to 50 mbar and finally to 11 mbar. The reaction was stopped after 4 days and the catalyst removed by vacuum filtration at 80°C. The polyester produced was a translucent viscous liquid. Mw(PEG-Standard) 4600, Polydispersity 3.3 and the acid value 33.1.

### Example 3

### Materials:

Mixed medium chain monounsaturated dicarboxylic acid, 25.00 g.
[Composition, main peaks in gas chromatography: C6:0 DCA-1.2%, C8:0 DCA-7.2%, C10:1 DCA-16 1%, C10:0 DCA-5.0%, C12.2 DCA-0.5%, C12:1 DCA-38.0%, C12:0 DCA-4.6%, C14:2-2.9%, C14:1 DCA-18.7%, produced by the fermentative method disclosed in WO 94/07837.]
1,4-butanediol, ex Fluka. 10.01 g.
Immobilised *Candida antarctica* B lipase, SP 435, ex Novo Nordisk, 1.00 g.

### Reaction:

The reaction was performed as in Example 1, except that the reaction temperature used was 70°C and the vacuum (100 mbar) was not applied until the end of the reaction at six days to remove condensates in the reactor vessel. The product was isolated by vacuum filtration at 70°C to give a yellow oil having an acid value of 71.6, Mw of 4800 and polydispersity 3.5.

### Example 4

### Materials:

9-octadecen 1,18-dioic acid, ex Unigema 25.00 g.
1,12-dodecanediol, ex Fluka, 14.98 g.
Immobilised *Candida antarctica B* lipase, SP 435 (Novo Nordisk, Denmark), 1.00 g.

### Reaction:

The reaction was performed as in Example 1, except that the reaction temperature used was 70°C, in order initially to melt the reaction mixture, and the vaccuum of 100 mbar was applied after 170 minutes and maintained until the end of the reaction at 22 hours. To remove catalyst from the products of the reaction a solvent, methylethylketone, was used to lower the viscosity and this slurry was vacuum-filtered at 70°C. The solvent was removed at 90°C under vacuum. The product was a wax and had an acid value of 23.5, Mw of 18500 and polydispersity 4.0.

### Example 5

### Materials:

9-octadecen 1.18-dioic acid, ex Unigema, 15.00 g.
1.12 dodecanedioic acid (C12:0 DCA), ex Aldrich, 9.905 g.
1,4-butanediol, ex Fluka, 7.752 g.
Immobilised *Candida antartica* B lipase. SP 435 (Novo Nordisk, Denmark), 1.00 g.

### Reaction:

The reaction was performed as in Example 1, except that the reaction temperature used was 70°C. Initially the reaction was started with only C18:1 DCA and the butanediol. After 1 hour of reaction, when the formation of some low molecular weight polymers and oligomers had occurred, the C12:0 DCA was added.. A vacuum of 100 mbar was slowly applied to the head space at 3 hours and maintained until the end of the reaction at 22.5 hours. After six hours the reaction mixture became homogeneous. The lipase catalyst was removed by vacuum-filtration at 70°C. The product was a soft wax at room temperature, and had an acid value of 23.5, Mw of 10400 and polydispersity 3.5.

### Example 6 - Measurement of antimicrobial properties

### Material:

*Coryneform (G42) bacterium:* is an isolate from human skin at Colworth Laboratories, Bedford, UK. It is a Gram +ve and lipase-producing organism. Corynebacteria are commonly found on human skin and are associated, amongst other things, with the causation of body malodour.
*Polyester emulsion:* the polyester prepared in Example 1, 1 g/l, was aseptically emulsified with 5 g/l Triton X100 in 10 mM, pH 6, Na-phosphate buffer.
*Growth medium:* 20 g/l CASO Bouillon, Merck: 10 g/l Yeast extract. Beta lab, UK; 2.5 g/l Tween 80, Sigma; Millipore water.
*Agar plates:* 30 g/l CASO Bouillon, Merck; 10 g/l Yeast extract, Beta lab. UK; 10 g/l Tween 80, Sigma: 20 g/l Agar, Oxoid; Millipore water.

### Methods:

*To test the effect of the made polyesters on Corynehacteria cells:* A suspension of freshly grown viable *Corynebacteria* cells were incubated in 30 ml polyester emulsion in shaking flasks at 35°C. Samples of 100 µl were taken at different times and Total Viable Count (TVC) of the cells was made. A cell suspension without polyester, and one with added free C18:1 dicarboxylic acid (at 0.5 gms/l), were treated in exactly the same way to act as negative and positive controls respectively.
*Total Viable Count:* After diluting the sample in Ringer's solution and making a dilution series, 100 µl from each dilution was applied and spread on to agar plates. These plates were then incubated at 35°C for two days or until the growing colonies were big enough to count.

### Results

**Table 2**

| **Total Viable Count (cells/ml)** | | | |
|---|---|---|---|
| Contact time (hours) | Cell control | Cells + polyester (MW 4,800) | Cells + free C18:1 DCA (0.5gms/l) |
| 0 | 2.5 x 10⁹ | 2.5 x 10⁹ | 2.5 x 10⁹ |
| 0.25 | - | - | 4.7 x 10⁸ |
| 1 | - | - | 1.0 x 10⁷ |
| 2 | - | 1.0 x 10⁸ | - |
| 5 | - | 6,000 | 40 |
| 8 | - | 110 | - |
| 24 | 3.3 x 10⁸ | 10 | 10 |

These results are also shown graphically in Figure 1. The data show that over a 24 hr period the number of viable cells incubated in the absence of polyester or free dioic acid was essentially stable, with less than a log decrease in cell number. In contrast, cell viability is essentially lost in the presence of either polyester or free dioic acid. In the presence of free acid, a >2 log reduction occurred within the first hour. In the presence of polyester (MW 4,800) there is a slower loss of viability (>1 log in 2 hours), presumably because the polyester must first be hydrolysed to give the active anti-microbial substance. However, the final viable count was virtually zero, as with the free acid-treated cells.

### Example 7 - Antimicrobial properties in presence or absence of added lipase

This study was performed essentially as in example 6 above.

### Result:

*Corynebacteria* were incubated with a polyester emulsion made with a C18:1 DCA-butanediol polyester with Mw=4800, as described in Example 1. In one test extra lipase was added (10 µl of Lipozyme 10 000L. Novo) together with the bacterial cells. The results are shown in Table 3 below, and graphically in Figure 2.

**Table 3**

| Effect of polyester emulsion on the TVC of *Corynebacteria* cells both without and with extra lipase added. | | |
|---|---|---|
| **Contact time (hrs)** | **TVC (viable cells/ml)** | |
| | **cells** | **cells + lipase** |
| 0 | 2.7 x 10⁹ | 3.4 x 10⁹ |
| 1 | 3.0 x 10⁹ | 4.1 x 10⁷ |
| 2 | 1.4 x 10⁹ | < 1 x 10⁴ |
| 4 | 1.0 x 10⁷ | < 1 x 10⁴ |
| 7 | < 1 x 10³ | < 1 x 10³ |
| 25.5 | < 1 x 10² | < 1 x 10² |

The addition of free lipase to the test system substantially increases the rate of loss of viability, due to the increased rate of hydrolysis of the polyester. This indicates that active compounds will he generated most rapidly at sites with high concentrations of lipase which, in turn, will be at sites of maximum microbial colonisation.

### Example 8 - Effect of polyesters of different molecular weight

This example relates to comparison of the effect of different molecular weight polyesters *on Corynebacteria* cells. This study was performed essentially as in Example 7. The polyesters used from Example 1 had Mw's of 4800, 16.600 and 34700. Additionally, the polyester from Example 2 with C18:1-glycerol was investigated in this example. The results are shown below in Table 4, and represented graphically in Figure 3.

**Table 4**

| Effect of different polyester emulsions on the TVC of Corynehacteria cells. | | | | |
|---|---|---|---|---|
| **Contact time (hrs).** | **butanediol polymer Mw = 4800** | **butanediol polymer Mw = 16600** | **butanediol polymer Mw = 34700** | **glycerol polymer Mw - "4600"** |
| 0 | 3.2 x 10⁹ | 3.2 x 10⁹ | 3.2 x 10⁹ | 3.2 x 10⁹ |
| 1 | 2.3 x 10⁹ | 3.0 x 10⁹ | 2.6 x 10⁹ | 3.1 x 10⁹ |
| 2 | 1.6 x 10⁹ | 3.2 x 10⁹ | 2.9 x 10⁹ | 2.2 x 10⁹ |
| 4 | 6.8 x 10⁷ | 0.96 x 10⁹ | 2.3 x 10⁹ | 7.5 x 10⁸ |
| 6 | 1.4 x 10⁵ | 1.5 x 10⁸ | 1.2 x 10⁹ | 7.5 x 10⁷ |
| 23 | < 10 | < 10 | < 10 | < 10 |

These data show that as the molecular weight of the C18:1 dioic acid/butanediol polyester is increased, the initial rate of antimicrobial activity is decreased because there is a lower concentration of "free ends" of polymer molecules available for lipase-mediated degradation of the free acid. The rate of loss of cell viability is lower with the 4,600 MW glycerol-based polyester than with the equivalent molecular weight butanediol-based polyester. This is presumably due to slower hydrolysis, which in turn is probably a result of steric hindrance problems caused by the presence of limited esterifieation at the secondary -OH group of the glycerol molecule. However, the polymer is still degradable.

Figure 3 shows the results for the initial 6 hours of the experiment. The final time point has been omitted from the graph to emphasise the difference in initial rates of hydrolysis (after 23 hours' incubation the cell viability is < 10 cells/ml for all the polymers used). Overall, the experiment shows that the nature of the polymeric molecule can be altered to affect the rate of release of the active substance.

### Example 9

This example investigates the effect of a polyester emulsion with mixed medium mono-unsaturated-DCA (with average molecular weight of about 4,800) from Example 3 on *Corynebacteria* cells. This study was performed as described in Example 5. The results are shown below in Table 5.

**Table 5**

| Effect of polyester emulsion on TVC of Corynebacteria cells. | |
|---|---|
| **Contact time (hrs)** | **Viable cells/ml** |
| 0 | 4.0 x 10⁹ |
| 1 | 2.3 x 10⁹ |
| 2 | 1.2 x 10⁹ |
| 5 | 4.5 x 10⁷ |
| 24 | 4.8 x 10³ |

## Claims

1. A polymeric molecule comprising a plurality of repeat units having the structure -(O₂C-R-CO₂)-, wherein R is a substituted or unsubstituted, C_{16:1}, C_{16:2}, C_{16:3} or a C_{20:1} hydrocarbon moiety, and wherein the molecule is a copolymer of a dioic acid esterified with glycerol, butanediol or a C₆-C₁₀ polyol.

2. A polymeric molecule according to claim 1, wherein R is aliphatic.

3. A polymeric molecule according to claim 1 or 2, wherein the polymer is lipase-degradable and, upon exposure to a lipase, releases a compound having an anti-microbial effect.

4. A polymeric molecule according to any one of the preceding claims having an average molecular weight in the range 3.5-10kDa.

5. A composition suitable for topical application to the human body, the composition comprising: a polymeric molecule having a plurality of repeat units having the structure - (O₂C-R-CO₂)-, wherein R is a substituted or unsubstituted, unsaturated hydrocarbon radical having 10 or more carbon atoms; and an acceptable carrier substance.

6. A composition according to claim 5, comprising a polymeric molecule in accordance with any one of claims 1 - 4.

7. A composition according to claim 5 or 6, comprising a plurality of different polymeric molecules, each polymeric molecule comprising a plurality of repeat units having the structure -(O₂C-R-CO₂)-, wherein R is a substituted or unsubstituted, unsaturated hydrocarbon radical having 13 or more carbon atoms.

8. A composition according to any one of claims 5-7, formulated for use as a pharmaceutical or cosmetic.

9. A composition according to any one of claims 5-8, being buffered to a pH in the range 4-7, preferably pH5.5-6.5.

10. A composition according to any one of claims 5-9, comprising oneor more of the following: a volatile organic solvent, an emollient, or an emulsifier.

11. A composition according to any one of claims 5-10, for use in the formation of a moisture-retentive, occlusive film.

12. A medical dressing for application to the skin of a human subject, the dressing comprising a dressing substrate impregnated or coated with a polymeric molecule according to any one of claims 1-4, or a composition according to any one of claims 5-11.

13. A method of treating the skin of a human subject, comprising applying to the skin a composition in accordance with any one of claims 5-11.

14. Use of a lipase-degradable polyester comprising a plurality of repeat units having the structure -(O₂C-R-CO₂)- wherein R is a substituted or unsubstituted, unsaturated hydrocarbon radical having 10 or more carbon atoms, with an acceptable carrier substance. in the manufacture of a pharmaceutical composition for the treatment of a human skin condition caused or exacerbated by, or associated with, microbial growth.

15. A method of making a cosmetic or pharmaceutical composition for topical application to a human subject, comprising mixing an effective amount of a polymer having a plurality of repeat units having the structure -(O₂C-R-CO₂)-, wherein R is a substituted or unsubstituted, unsaturated hydrocarbon radical having 10 or more carbon atoms, with an acceptable carrier substance.

16. A method according to claim 15, wherein the composition comprises a polymeric molecule in accordance with any one of claims 1-4.

## Patentansprüche

1. Polymeres Molekül, das eine Vielzahl an Grundeinheiten mit der Struktur-(O₂C-R-CO₂)- umfasst, wobei R eine substituierte oder unsubstituierte C_{16:1}-, C_{16:2}-, C_{16:3}oder ein C_{20:1}-Kohlenwasserstoffeinheit ist, und wobei das Molekül ein Copolymer einer Dicarbonsäure ist, die mit Glycerin, Butandiol oder einem C₆-C₁₀-Polyol verestert ist.

2. Polymeres Molekül nach Anspruch 1, wobei R aliphatisch . ist.

3. Polymeres Molekül nach Anspruch 1 oder 2, wobei das Polymer durch Lipase zersetzbar ist und unter Einwirkung einer Lipase eine Verbindung mit einer anti-mikrobiellen Wirkung freisetzt.

4. Polymeres Molekül nach einem der vorhergehenden Ansprüche mit einem durchschnittlichen Molekulargewicht im Bereich von 3,5-10 kDa.

5. Zusammensetzung, die zur topischen Anwendung auf den menschlichen Körper geeignet ist, wobei die Zusammensetzung umfasst: Ein polymeres Molekül, das eine Vielzahl an sich wiederholenden Einheiten mit der Struktur-(O₂C-R-CO₂)- hat, wobei R ein substituierter oder unsubstituierter, ungesättigter Kohlenwasserstoffrest mit 10 oder mehr Kohlenstoffatomen ist; und eine akzeptable Trägersubstanz.

6. Zusammensetzung nach Anspruch 5, die ein polymeres Molekül gemäß einem der Ansprüche 1-4 umfasst.

7. Zusammensetzung nach Anspruch 5 oder 6, die eine Vielzahl verschiedener polymerer Moleküle umfasst, wobei jedes polymere Molekül eine Vielzahl an sich wiederholenden Einheiten mit der Struktur-(O₂C-R-CO₂)-umfasst, wobei R ein substituiertes oder unsubstituiertes, ungesättigtes Kohlenwasserstoffradikal mit 13 oder mehr Kohlenstoffatomen ist.

8. Zusammensetzung nach einem der Ansprüche 5-7, die zur Verwendung als ein Pharmazeutikum oder Kosmetikum formuliert ist.

9. Zusammensetzung nach einem der Ansprüche 5-8, die auf einen pH-Wert in dem Bereich von 4-7, vorzugsweise pH 5,5-6,5, gepuffert ist.

10. Zusammensetzung nach einem der Ansprüche 5-9, die eines oder mehrere der folgenden umfasst: ein flüchtiges organisches Lösungsmittel, einen Weichmacher, oder einen Emulgator.

11. Zusammensetzung nach einem der Ansprüche 5-10 zur Verwendung bei der Bildung eines feuchtigkeitsrückhaltenden, okkludierenden Films.

12. Medizinischer Verbandsstoff zur Anwendung auf der Haut einer menschlichen Person, wobei der Verbandsstoff ein Verbandsubstrat umfasst, das mit einem polymeren Molekül gemäß einem der Ansprüche 1-4 oder mit einer Zusammensetzung nach einem der Ansprüche 5-11 imprägniert oder überzogen ist.

13. Verfahren zur Behandlung der Haut einer menschlichen Person, das die Anwendung einer Zusammensetzung gemäß einem der Ansprüche 5-11 auf die Haut umfasst.

14. Verwendung eines Polyesters, der durch Lipase zersetzbar ist und eine Vielzahl an sich wiederholenden Einheiten mit der Struktur -(O₂C-R-CO₂)- umfasst, wobei R ein substituierter oder unsubstituierter, ungesättigter Kohlenwasserstoffrest mit 10 oder mehr Kohlenstoffatomen ist, mit einer akzeptablen Trägersubstanz, bei der Herstellung einer pharmazeutischen Zusammensetzung für die Behandlung einer menschliche Hautbeschaffenheit, die durch mikrobielles Wachstum verursacht oder verschlimmert wurde oder damit zusammenhängt.

15. Verfahren zur Herstellung einer kosmetischen oder pharmazeutischen Zusammensetzung zur topischen Anwendung auf eine menschliche Person, das das Vermischen einer wirksamen Menge eines Polymers, das eine Vielzahl an sich wiederholenden Einheiten mit der Struktur -(O₂C-R-CO₂)-hat, wobei R ein substituierter oder unsubstituierter, ungesättigter Kohlenwasserstoffrest mit 10 oder mehr Kohlenstoffatomen ist, mit einer akzeptablen Trägersubstanz umfasst.

16. Verfahren nach Anspruch 15, wobei die Zusammensetzung ein polymeres Molekül gemäß einem der Ansprüche 1-4 umfasst.

## Revendications

1. Molécule polymère comprenant une pluralité d'unités répétitives ayant la structure -(O₂C-R-CO₂)-, dans laquelle R est un radical hydrocarboné substitué ou non substitué en C_{16:1}, C_{16:2}, C_{16:3} ou C_{20:1}, et dans lequel la molécule est un copolymère d'un acide dioïque estérifié avec un glycérol, un butane diol ou un polyol en C₆-C₁₀.

2. Molécule polymère selon la revendication 1, dans laquelle R est aliphatique.

3. Molécule polymère selon la revendication 1 ou 2, dans laquelle le polymère est dégradable par lipase et, lors de l'exposition à une lipase, il libère un composé ayant un effet anti-microbien.

4. Molécule polymère selon une quelconque des revendications précédentes, ayant un poids moléculaire moyen dans la plage de 3,5 à 10 kDa.

5. Composition convenant pour une application topique au corps humain, la composition comprenant : une molécule polymère ayant une pluralité d'unités répétitives de structure -(O₂C-R-CO₂)-, dans laquelle R est un radical hydrocarboné insaturé, substitué ou non substitué, ayant 10 atomes de carbone ou plus, et une substance porteuse acceptable.

6. Composition selon la revendication 5, comprenant une molécule polymère selon une quelconque des revendications 1 à 4.

7. Composition selon la revendication 5 ou 6, comprenant une pluralité de molécules polymères différentes, chaque molécule polymère comprenant une pluralité d'unités répétitives ayant la structure -(O₂C-R-CO₂)-, dans laquelle R est un radical hydrocarboné insaturé, substitué ou non substitué, ayant 13 atomes de carbone ou plus.

8. Composition selon une quelconque des revendications 5 à 7, formulée pour une utilisation comme produit pharmaceutique ou cosmétique.

9. Composition selon une quelconque des revendications 5 à 8, tamponnée à un pH dans la plage de 4 à 7, et de préférence un pH de 5,5 à 6,5.

10. Composition selon une quelconque des revendications 5 à 9, comprenant une ou plusieurs des substances suivantes : un solvant organique volatil, un émollient ou un émulsifiant.

11. Composition selon une quelconque des revendications 5 à 10, utilisable dans la formation d'un film occlusif de rétention d'humidité.

12. Pansement médical pour application à la peau d'un sujet humain, le pansement comprenant un substrat de pansement imprégné ou revêtu avec une molécule polymère selon une quelconque des revendications 1 à 4, ou une composition selon une quelconque des revendications 5 à 11.

13. Procédé de traitement de la peau d'un sujet humain, comprenant l'application à la peau d'une composition selon une quelconque des revendications 5 à 11.

14. Utilisation d'un polyester dégradable par lipase, comprenant une pluralité d'unités répétitives ayant la structure -(O₂C-R-CO₂)-, dans laquelle R est un radical hydrocarboné insaturé, substitué ou non substitué, ayant 10 atomes de carbone ou plus, avec une substance porteuse acceptable, dans la fabrication d'une composition pharmaceutique pour le traitement d'un état de la peau humaine provoqué ou aggravé par un développement microbien ou associé à ce dernier.

15. Procédé de fabrication d'une composition cosmétique ou pharmaceutique pour application topique à un sujet humain, comprenant le mélange d'une quantité efficace d'un polymère ayant une pluralité d'unités répétitives de structure -(O₂C-R-CO₂)-, dans laquelle R est un radical hydrocarboné insaturé, substitué ou non substitué, ayant 10 atomes de carbone ou plus, avec une substance porteuse acceptable.

16. Procédé selon la revendication 15, dans lequel la composition comprend une molécule polymère selon une quelconque des revendications 1 à 4.
